# EUROPEAN PATENT SPECIFICATION

(11) **EP 1 443 326 B1**
(45) Date of publication and mention of the grant of the patent: **23.02.2011**
(21) Application number: 02775332.6
(22) Date of filing: 11.10.2002
(51) Int. Cl.: G01N 33/53, G01N 33/577, G01N 33/68

(54) **METHOD OF DIAGNOSING CEREBRAL INFARCTION**
VERFAHREN ZUR DIAGNOSE EINES HIRNINFARKTS
PROCEDE DE DIAGNOSTIC D'UN INFARCTUS CEREBRAL

(30) Priority: 11.10.2001 JP 2001314357
(43) Date of publication of application: 04.08.2004
(73) Proprietor: Mitsubishi Chemical Medience Corporation, 2-8, Shibaura 4-chome Minato-ku Tokyo 108-8559 (JP)
(72) Inventor: KOIKE, Takao, Sapporo-shi, Hokkaido 064-0952 (JP); ATSUMI, Tatsuya, Sapporo-shi, Hokkaido 001-0905 (JP); KATO, Hisao, Suita-shi, Osaka 565-0872 (JP); TANAKA, Hideyuki, Tokyo 108-8559 (JP)
(74) Representative: Vossius & Partner
(86) International application number: PCT/JP2002/010610
(87) International publication number: WO 2003/034066

(56) References cited:
- WO-A1-95/09363
- JP-A- 2000 028 607
- CHEN W H ET AL: "An unusual increase of blood anti-beta 2-glycoprotein-I antibody but not antiphospholipid antibody in cerebral ischemia--a case report." ANGIOLOGY FEB 2001, vol. 52, no. 2, February 2001 (2001-02), pages 149-154, XP009085863 ISSN: 0003-3197
- HORBACH D A ET AL: "Beta2-glycoprotein I is proteolytically cleaved in vivo upon activation of fibrinolysis." THROMBOSIS AND HAEMOSTASIS JAN 1999, vol. 81, no. 1, January 1999 (1999-01), pages 87-95, XP009085825 ISSN: 0340-6245
- HUNT J.E. ET AL. PROC. NATL. ACAD. SCI. USA vol. 90, 1993, pages 2141 - 2145, XP002961064
- OHKURA N. ET AL. BLOOD vol. 91, no. 11, 1998, pages 4173 - 4179, XP002961065

## Description

### TECHNICAL FIELD

The present invention relates to a method of diagnosing cerebral infarction. Particularly, the present invention may be used for the diagnosis of patients suffering from cerebral infarction and the observation of prognosis after treatment.

The term "nicked β2 glycoprotein I" as used herein means β2 glycoprotein I, cleaved by a protease at the one peptide bond and converted into the two polypeptide chains linked to each other by disulfide bonds.

When β2 glycoprotein I not cleaved by the protease is to be distinguished from the "nicked β2 glycoprotein I", it is referred as "intact β2 glycoprotein I". The term "β2 glycoprotein I" as used herein means the "intact β2 glycoprotein I", unless otherwise specified.

The term "total β2 glycoprotein I" as used herein includes both the "nicked β2 glycoprotein I" and the "intact β2 glycoprotein I"

### BACKGROUND ART

β2 glycoprotein I is a glycoprotein and its plasma concentration is approximately 200 µg/mL in healthy person. It is known to bind negatively charged phospholipids. For example, it is supposed to show anticoagulant activity by regulate the interaction between protein S and its binding protein, activated factor V, a phospholipid-dependent prothrombinase activity, platelet aggregation by ADP, and contact of intrinsic coagulation factors to phospholipid phase.

Further, it has been reported that the affinity of nicked β2 glycoprotein I cleaved by the protease for anionic phospholipids was decreased to approximately 1/100 or less of that of intact β2 glycoprotein I [J. E. Hunt, Proc. Natl. Acad. Sci. USA, Vol. 90, p. 2141-2145 (1993)]. In various diseases, after the intravascular coagulation pathway is activated, a fibrinolytic enzyme, plasmin, is activated, and then β2 glycoprotein I is cleaved by plasmin [Ohkura et al., Blood, Vol. 91, p. 4173-4179 (1998)]. It is suggested that the affinity of nicked β2 glycoprotein I for anionic phospholipids is decreased by the cleavage and then nicked β2 glycoprotein I is released into the blood flow.

Cerebral infarction is caused by an obstruction in cerebral arteries, which is caused by thrombi. It is the major type of cerebral apoplexy with a high mortality and the third frequent cause of death in Japan.

Diagnostic imaging such as a CT examination has been used in the diagnosis of cerebral infarction so far. If a specific marker for cerebral infarction can be measured besides the CT examination, it would be very useful in the diagnosis or prognosis of cerebral infarction.

Angiology 52 (2) 2001, pages 149-154 describes that anti-B2 glycoprotein can be used as marker for cerebral ischemia.

### DISCLOSURE OF THE INVENTION

The present inventors have intensively studied to develop of a method for diagnosing (i.e., detecting) cerebral infarction. As a result, they found that the amounts of nicked β2 glycoprotein I in patients with cerebral infarction are significantly higher than those in healthy person, and that the ratios of the nicked β2 glycoprotein I concentration to the total β2 glycoprotein I concentration are useful for the diagnosis of cerebral infarction.

Conventional molecular markers for coagulation and fibrinolysis, such as a TAT (thrombin-antithrombin III complex) value, a PIC (plasmin-α2 plasmin inhibitor complex) value, and a D/D (D dimer) value, showed no significant difference between healthy persons and patients with cerebral infarction (or patients with a history of cerebral infarction). However, amounts of nicked β2 glycoprotein I (for example, nicked β2 glycoprotein I cleaved by plasmin) in patients with cerebral infarction are significantly higher than those in healthy persons, and the ratios of the nicked β2 glycoprotein I concentration to the total β2 glycoprotein I concentration in patients with cerebral infarction are significantly higher than those in healthy persons. Therefore, nicked β2 glycoprotein I and the ratio of the nicked β2 glycoprotein I concentration to the total β2 glycoprotein I concentration can be a sensitive marker useful for the diagnosis of cerebral infarction.

The present invention is based on the above findings.

The present invention relates to a method of diagnosing cerebral infarction, comprising the step of: measuring a concentration of nicked β2 glycoprotein in a body fluid sample.

The present invention also relates to a method of diagnosing cerebral infarction, comprising the steps of: measuring a nicked β2 glycoprotein I concentration (N) and a total β2 glycoprotein I concentration (T) in a body fluid sample, calculating a ratio (N/T) of the nicked β2 glycoprotein I concentration (N) to the total β2 glycoprotein I concentration (T), and using the ratio as an indicator.

In a preferred embodiment of the present invention, their concentrations are measured by immunological methods or biochemical methods.

The term "diagnosis of cerebral infarction" as used herein mainly means the detection or prognosis of cerebral infarction, and, in a broad sense, includes the screening or monitoring of symptoms during or after treatment.

The term "nicked β2 glycoprotein I" as used herein means β2 glycoprotein I, which is cleaved by the protease at the one peptide bond and converted into two polypeptide chains linked to each other by disulfide bonds, as described above. More particularly, "nicked β2 glycoprotein I" includes, for example, (1) nicked β2 glycoprotein I in which the domain V is cleaved by plasmin (human β2 glycoprotein I is cleaved at the peptide bond between the 317th lysine and the 318th threonine) and (2) nicked β2 glycoprotein I in which the domain V is cleaved by granulocyte elastase (human β2 glycoprotein I is cleaved at the peptide bond between the 314th alanine residue and the 315th phenylalanine residue).

### BRIEF DESCRIPTION OF THE DRAWINGS

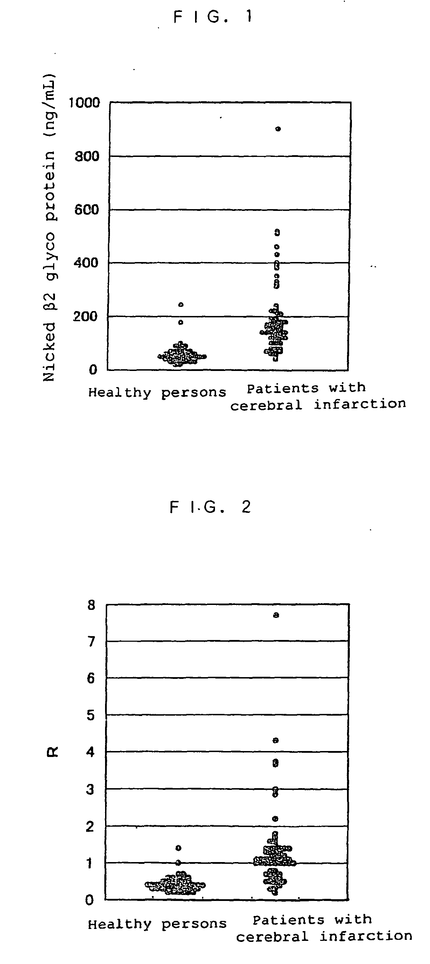

Figure 1 is a bar graph separately shows concentrations of the nicked β2 glycoprotein I (nicked β2GPI) in plasma samples from healthy persons and from patients with cerebral infarction, measured by a sandwich enzyme immunoassay according to the method of the present invention.
Figure 2 is a bar graph separately shows ratios (R) calculated from nicked β2 glycoprotein I concentrations and total β2 glycoprotein I concentrations in plasma samples from healthy persons and from patients with cerebral infarction, measured by a sandwich enzyme immunoassay according to the method of the present invention.

### BEST MODE FOR CARRYING OUT THE INVENTION

According to the method of the present invention, cerebral infarction can be diagnosed with a high sensitivity. Cerebral infarction is caused by an obstruction in cerebral arteries, which is caused by thrombi. Cerebral circulation disorder is caused by constriction or obstruction in extracranial or intracranial cerebral arteries. As a result, the brain tissue is irreversibly damaged and shows a cerebral infarction. Various neurologic symptoms are observed from the damaged brain tissue. On the basis of a mechanism of the generated vascular obstruction, cerebral infarction is classified into embolic cardiogenic cerebral infarction or thrombotic lacunar infarction, or atherothrombotic cerebral infarction to which sharing stress is added. The diagnosis thereof is carried out by diagnostic imaging (Computed Tomography, Magnetic Resonance Angiography) findings and clinical findings (neurologic signs or the like) [Higashi et al., Medical Technology, Vol. 29, No. 2, p. 138-146 (2001)]. Further, as an auxiliary approach to the diagnosis of cerebral infarction (lacunar infarction), measurement of a TAT (thrombin-antithrombin III complex) value, a PIC (plasmin-α2 plasmin inhibitor complex) value, or a DD (D-D dimer) value is carried out as a coagulation test [Yamaguchi et al., "Kyou no Sindan-Shishin" the third edition, Igaku-Syoin, p. 499-500 (1992)].

The term "cerebral infarction" as used herein means diseases or symptoms diagnosed by the above-described diagnosis methods and includes diseases or symptoms during the observation period of treatment after cerebral infarction.

In the method of the present invention, any body fluid sample from any mammal (particularly a human) is available. For a body fluid sample, for example, blood samples such as plasma or serum, plasma is preferred, especially.

The present inventors found that the concentrations of nicked β2 glycoprotein I in body fluid samples from patients with cerebral infarction are significantly higher than those from healthy persons, as described in Examples.

Therefore, in the method of the present invention, cerebral infarction can be diagnosed by measuring the concentration of nicked β2 glycoprotein I in body fluid samples collected from subjects to be tested.

The concentration of nicked β2 glycoprotein I can be measured by, for example, an immunological method or a biochemical method.

Further, the present inventors found that the ratio (N/T, i.e., R value) of the nicked β2 glycoprotein I concentration (N) to the total β2 glycoprotein I concentration (T) in body fluid samples from patients with cerebral infarction is significantly higher than that from healthy persons, as described in Examples.

Therefore, in the method of the present invention, cerebral infarction can be diagnosed by measuring the nicked β2 glycoprotein I concentration (N) and the total β2 glycoprotein I concentration (T) in body fluid samples collected from subjects to be tested, calculating the ratio (N/T, i.e., R value) of the nicked β2 glycoprotein I concentration (N) to the total β2 glycoprotein I concentration (T), and using the R value as an indicator.

The concentration of nicked β2 glycoprotein I can be measured by, for example, the above immunological method or biochemical method. Similarly, the concentration of total β2 glycoprotein I can be measured by the immunological method or biochemical method.

The immunological measurement of nicked β2 glycoprotein I can be carried out by using, for example, a "monoclonal antibody which does not react to intact β2 glycoprotein I but reacts to nicked β2 glycoprotein I" (hereinafter sometimes referred to as an anti-nicked β2 GPI monoclonal antibody) described in Japanese Unexamined Patent Publication (Kokai) No. 2000-28607, or an antibody fragment thereof.

As the anti-nicked β2 GPI monoclonal antibody, there may be mentioned, preferably (1) an antibody which does not react to intact β2 glycoprotein I but reacts to nicked β2 glycoprotein I and the domain V of nicked β2 glycoprotein I [particularly an antibody in which the epitope is located in the region consisting of the 242nd to 326th amino acid residues (numbered from the amino-terminus) in human β2 glycoprotein I] (more preferably, monoclonal antibody NGPI-59 secreted from hybridoma NGPI-59) or (2) an antibody which does not react to intact β2 glycoprotein I but reacts to nicked β2 glycoprotein I and a region consisting of the domains I to IV of nicked β2 glycoprotein I [particularly an antibody in which the epitope is located in the region consisting of the 1st to 241st amino acid residues (numbered from the amino-terminus) in human β2 glycoprotein I] (more preferably, monoclonal antibody NGPI-60 secreted from hybridoma NGPI-60).

The immunological measurement of total β2 glycoprotein I can be carried out by using, for example, a "monoclonal antibody which reacts to intact β2 glycoprotein I and nicked β2 glycoprotein I" (hereinafter sometimes referred to as an anti-total β2 GPI monoclonal antibody) described in Japanese Unexamined Patent Publication (Kokai) No. 2000-28607, or an antibody fragment thereof.

As the anti-total β2 GPI monoclonal antibody, an antibody which reacts to intact β2 glycoprotein I (particularly human intact β2 glycoprotein I) and nicked β2 glycoprotein I (particularly human nicked β2 glycoprotein I in which the domain V is cleaved), and preferably in which the epitope is located in the region consisting of the domains I to IV of intact β2 glycoprotein I and nicked β2 glycoprotein I [for example, the region consisting of the 1st to 241st amino acid residues (numbered from the amino-terminus) in human β2 glycoprotein I], is preferable, and monoclonal antibody NGPI-23 secreted from hybridoma NGPI-23 is more preferable.

The hybridomas NGPI-23(FERM BP-8202), NGPI-59(FERM BP-8203), and NGPI-60(FERM BP-8204) were domestically deposited in the International Patent Organism Depositary National Institute of Advanced Industrial Science and Technology [(Former Name) National Institute of Bioscience and Human-Technology Agency of Industrial Science and Technology (Address: AIST Tsukuba Central 6, 1-1, Higashi 1-chome Tukuba-shi, Ibaraki-ken 305-8566 Japan)] on July 9, 1998, and transferred to an international deposit on October 11, 2002. The international deposit numbers (a number in parenthesis [] following the international deposit number is a domestic deposit number) are FERM BP-8202[FERM P-16891], FERM BP-8203[FERM P-16892], and FERM BP-8204[FERM P-16893], respectively.

As the antibody fragment of the anti-nicked β2 GPI monoclonal antibody or the anti-total β2 GPI monoclonal antibody, a fragment of each monoclonal antibody with the same specificity as that of the original monoclonal antibody can be used. The antibody fragments include, or example, Fab, Fab', F(ab')₂, or Fv.

In the immunological measurement of nicked β2 glycoprotein I, an amount of nicked β2 glycoprotein I in a body fluid sample can be measured, for example, by immobilizing an anti-nicked β2 GPI monoclonal antibody (or an antibody fragment thereof) on an insoluble carrier as a first antibody; bringing the immobilized first antibody into contact with the body fluid sample; bringing the resulting complex into contact with a labeled anti-nicked β2 GPI monoclonal antibody (or a fragment thereof) different from the first antibody or a labeled anti-total β2 GPI monoclonal antibody (or a fragment thereof), as a second antibody; and detecting a signal from the labeled second antibody which binds to the immobilized first antibody-nicked β2 glycoprotein I complex, or a signal from the labeled second antibody which does not bind to the immobilized first antibody-nicked β2 glycoprotein complex (sandwich method).

Alternatively, an amount of nicked β2 glycoprotein I in a body fluid sample can be measured, for example, by immobilizing at least an anti-nicked β2 GPI monoclonal antibody (or an antibody fragment), and another anti-nicked β2 GPI monoclonal antibody (or a fragment thereof) or a anti-total β2 GPI monoclonal antibody (or a fragment thereof) on an insoluble carrier; and bringing the immobilized monoclonal antibody (or the antibody fragment thereof) into contact with the body fluid sample (aggregation method). In this case, an aggregation reaction with intact β2 glycoprotein I in the body fluid sample does not occur, and only an aggregation reaction with nicked β2 glycoprotein I occurs.

As described above, in the immunological analysis of nicked β2 glycoprotein I, even if a body fluid sample is used without pretreatment (for example, separation of intact β2 glycoprotein I and nicked β2 glycoprotein I by chromatography), an interference of intact β2 glycoprotein I in the body fluid sample can be avoided.

In the immunological analysis of nicked β2 glycoprotein I utilizing the sandwich method, more particularly, an anti-nicked β2 GPI monoclonal antibody (such as the above-described monoclonal antibody NGPI-59 or NGPI-60) or an antibody fragment thereof is immobilized on an appropriate insoluble carrier (first antibody). The surface of the insoluble carrier is coated with an appropriate blocking agent, such as bovine serum albumin (BSA) or gelatin, to avoid a nonspecific binding of a body fluid sample to the insoluble carrier. An undiluted body fluid sample is added and brought into contact with the insoluble carrier, and the reaction is carried out at a constant temperature (for example, 4-40°C, preferably around room temperature) for a fixed time (for example, 5 minutes to 3 hours) (first reaction). Then, a labeled anti-nicked β2 GPI monoclonal antibody (or a fragment thereof) different from the monoclonal antibody used as the first antibody, or a labeled anti-total β2 GPI monoclonal antibody, such as monoclonal antibody NGPI-23, (or a fragment thereof), as the second antibody, is added, and the reaction is carried out at a constant temperature (for example, 4-40°C, preferably around room temperature) for a fixed time(for example, 5 minutes to 3 hours) (second reaction). The resulting insoluble carrier is washed with an appropriate washing liquid, such as saline containing a detergent, and then an amount of the labeled antibody on the insoluble carrier is measured. An amount of nicked β2 glycoprotein I in the body fluid sample can be calculated from the measured value. Alternatively, the first reaction and the second reaction can be carried out simultaneously.

The immunological measurement of total β2 glycoprotein I can be carried out in a manner similar to the immunological measurement of nicked β2 glycoprotein I, except that the anti-total β2GPI monoclonal antibody or an antibody fragment thereof is used instead of the anti-nicked β2GPI monoclonal antibody or an antibody fragment thereof (sandwich method).

The insoluble carrier which may be used in the immunological analysis utilizing the sandwich method is not particularly limited. The other insoluble carrier, for example, polymers (such as polyethylene, polystyrene, polypropylene, polyvinylchloride, polyester, polyacrylonitrile, fluororesin, crosslinked dextran, or polysaccharide), nitrocellulose, paper, or agarose, or a combination thereof, are also available. As the labeled substance, enzymes, fluorescent substances, or luminescent substances are preferred. For example, alkaline phosphatase, peroxidase, β-D-galactosidase, or the like, as the enzymes; fluorescein isothiocyanate or the like, as the fluorescent substances; and acridinium esters, luciferin, or the like, as the luminescent substances, are available.

As the insoluble carrier in the immunological analysis utilizing the aggregation reaction, an insoluble carrier commonly used in the immunological analysis utilizing the aggregation of an antigen-antibody reaction can be used. As the insoluble carrier, there may be mentioned, for example, latex particles (particularly polystyrene latex particles). To immobilize the monoclonal antibody on the insoluble carrier, known methods, such as a chemically binding method, in which carbodiimide, glutaraldehyde, or the like is used as a crosslinking agent, or a physically adsorption method can be used.

The concentrations of nicked β2 glycoprotein I and total β2 glycoprotein I can be measured by, for example, a biochemical method. For example, a biochemical measurement of nicked β2 glycoprotein I and intact β2 glycoprotein I can be carried out by fractionating human plasma treated with perchloric acid by an affinity chromatography using a heparin column such as a HiTrap-Heparin column (Pharmacia) [Ohkura et al., Blood, Vol. 91, p. 4173-4179 (1998)]. In this method, nicked β2 glycoprotein I and intact β2 glycoprotein I can be separated by the difference of the eluted position thereof (i.e., the difference of affinity for heparin). Each amount of nicked β2 glycoprotein I and intact β2 glycoprotein I can be measured by performing a determination of proteins in the eluted fractions. The concentration of total β2 glycoprotein I can be calculated from each amount of nicked β2 glycoprotein I and intact β2 glycoprotein I, and then the ratio (N/T=R) of the nicked β2 glycoprotein I concentration (N) to the total β2 glycoprotein I concentration (T) can be calculated from these values.

### EXAMPLES

The present invention now will be further illustrated by, but is by no means limited to, the following Examples.

### EXAMPLE 1: Measurement of nicked β2 glycoprotein I

In this example, the concentrations of nicked β2 glycoprotein I in the groups of patients with cerebral infarction and healthy persons were measured. In this connection, the patients with cerebral infarction were those who had experienced cerebral infarction and were regularly followed up.

Plasmas collected from the patients with cerebral infarction and from the healthy persons were used as samples, and nicked β2 glycoprotein I was measured by the following procedures.

To each well in a 96-well microtiter plate for ELISA (Immulon-II; Nihon Dynatec KK), 100 µL of Tris buffer A [50 mmole/L Tris-HCl, 0.15 mole/L NaCl(pH7.5)] containing 10 µg/mL of the fragment F(ab')₂ of the monoclonal antibody NGPI-60 was added and allowed to stand at 4°C for 18 hours. The plate was washed three times with a washing liquid W (0.05% Tween-20, 0.5 mole/L NaCl). Into wells of the antibody-immobilized plate, 100 µL of standard samples prepared by adding nicked β2 glycoprotein I into Tris buffer B [20 mmol/L Tris-HCl, 0.5 mole/L NaCl, 0.05% Tween-20 (pH7.6)] at concentrations of 200 ng/mL, 100 ng/mL, 50 ng/mL, 25 ng/mL, 12.5 ng/mL, and 6.25 ng/mL, and 100 µL of assay samples prepared by diluting plasmas with Tris buffer B were added and reacted at 25°C for 2 hours.

The plate was washed three times with the washing liquid W (0.05% Tween-20, 0.5 mole/L NaCl), and then 100 µL of the Tris buffer B [20 mmol/L Tris-HCl, 0.5 mole/L NaCl, 0.05% Tween-20 (pH7.6)] containing 2 µg/mL of the fragment F(ab')₂ of the biotin-labeled monoclonal antibody NGPI-23 was added to each well, and reacted at 25°C for an hour. Each well was washed three times with the washing liquid W (0.05% Tween-20, 0.5 mole/L NaCl), 100 µL of peroxidase-labeled avidin (DAKO) diluted 2000 times with the Tris buffer B was added to each well, and reacted at 25°C for an hour. Each well was washed three times with the washing liquid W, and 200 µL of an enzyme substrate liquid [10 mmole/L phenol/0.5 mmole/L 4-aminoantipyrine/0.005% hydrogen peroxide-containing 50 mmole/L Tris-HCl, 0.15 mole/L NaCl (pH7.5)] was added to each well. The absorbance at 492 nm of each well was measured by a microplate reader (MPR A4i type; Tosoh Corporation). A calibration curve was made from the absorbance in each concentration of the standard samples, and the concentrations of nicked β2 glycoprotein I in the plasma samples were determined from the calibration curve.

The results are shown in Table 1 and Figure 1. Values "average ± SD" of the concentration of nicked β2 glycoprotein I were 58.60 ± 38.98 ng/mL (group of healthy persons, N=44) and 196.69 ± 145.75 ng/mL (group of patients with cerebral infarction, N=63), respectively. A significant difference (p<0.001) of the group of patients with cerebral infarction to that of healthy persons was observed.

**Table 1**

| | healthy person | cerebral infarction patients |
|---|---|---|
| conc. (ng/mL) | 58.60 ± 38.98 | 196.69 ± 145.75 |

### Significant difference

healthy person vs cerebral infarction: p<0.001

### Example 2: Measurement of total β2 glycoprotein I and R value

In this example, total β2 glycoprotein I in the groups of patients with cerebral infarction and healthy persons was measured, and the R value were calculated.

Plasmas used in Example 1 were used as samples, and total β2 glycoprotein I was measured by the following procedures.

To each well in a 96-well microtiter plate for ELISA (Immulon-II; Nihon Dynatec KK), 50 µL of Tris buffer A [50 mmole/L Tris-HCl, 0.15 mole/L NaCl(pH7.5)] containing 5 µg/mL of the monoclonal antibody NGPI-23 was added and allowed to stand at 4°C for 18 hours. The plate was washed three times with the washing liquid W (0.05% Tween-20, 0.5 mole/L NaCl). Into wells of the antibody-immobilized plate, 50 µL of standard samples prepared by adding intact β2 glycoprotein I into Tris buffer B [20 mmole/L Tris-HCl, 0.5 mole/L NaCl, 0.05% Tween-20 (pH7.6)] at concentrations of 200 ng/mL, 100 ng/mL, 50 ng/mL, 25 ng/mL, 12.5 ng/mL, and 6.25 ng/mL, and 50 µL of assay samples prepared by diluting plasmas with the Tris buffer B were added and reacted at 25°C for 2 hours.

The plate was washed three times with the washing liquid W (0.05% Tween-20, 0.5 mole/L NaCl), and then 50 µL of the Tris buffer B [20 mmole/L Tris-HCl, 0.5 mole/L NaCl, 0.05% Tween-20 (pH7.6)] containing 2.5 µg/mL of a biotin-labeled anti-human β2 glycoprotein I rabbit polyclonal antibody (CEDARLANE) was added to each well, and reacted at 25°C for an hour. Each well was washed three times with the washing liquid W (0.05% Tween-20, 0.5 mole/L NaCl), 100 µL of peroxidase-labeled avidin (Dako) diluted 2000 times with the Tris buffer B was added to each well, and reacted at 25°C for an hour. Each well was washed three times with the washing liquid W, and 200 µL of an enzyme substrate solution [10 mmole/L phenol/0.5 mmole/L 4-aminoantipyrine/0.005% hydrogen peroxide-containing Tris buffer A [50 mmole/L Tris-HCl, 0.15 mole/L NaCl (pH7.5)]] was added to each well. The absorbance at 492 nm of each well was measured by a microplate reader (MPR A4i type; Tosoh Corporation). A calibration curve was made from the absorbance in each concentration of the standard samples, and the concentrations of total β2 glycoprotein I in the plasma samples were determined from the calibration curve.

Using the nicked β2 glycoprotein I concentration (N) measured in Example 1, each ratio (N/T=R) of the nicked β2 glycoprotein I concentration (N) to the total β2 glycoprotein I concentration (T) measured in this Example was calculated for each sample. The results are shown in Table 2 and Figure 2. A significant difference (p<0.001) of the group of patients with cerebral infarction to that of healthy persons was observed.

**Table 2**

| | healthy person | cerebral infarction patients |
|---|---|---|
| R | 0.44±0.22 | 1.30±1.15 |

### Significant difference

healthy person vs cerebral infarction: p<0.001

### Example 3: Test for correlation between TAT, PIC, and DD measured values and nicked β2 glycoprotein I value

For the group of patients with infarction, a TAT (thrombin-antithrombin III complex) value, a PIC (plasmin-α2 plasmin inhibitor complex) value, and a DD (D-D dimer) value were measured by the conventional methods in this example. The correlation between these values and the nicked β2 glycoprotein I value were examined.

In this example, the plasmas used in Examples 1 and 2 were used. The coagulation and fibrinolysis markers including TAT, PIC, and DD were measured by an aggregation method (Iatron Laboratories, Inc.).

As a result, the correlation to nicked β2 glycoprotein I were as follows:
TAT value: r = 0.017,
PIC value: r = -0.028,
DD value: r = -0.018.

No correlation between each value and nicked β2 glycoprotein I was observed.

### Example 4: Comparison of TAT, PIC, and DD values in the group of patients with cerebral infarction to normal standard values

Using the TAT, PIC, and DD values measured in Example 3, these values were compared to the normal standard values (referential standard values described in documents attached to each reagent). The results are shown in Table 3.

**Table 3**

| | standard values | cerebral infarction patients |
|---|---|---|
| TAT | < 3 ng/mL | 1.87±3.59 ng/mL |
| DD | < 1 µg/mL | 0.84±1.34 µg/mL |
| PPI | < 0.8 µg/mL | 1.36±1.02 µg/mL |

As a result, little significant difference between TAT, PIC, and DD values in the group of patients with cerebral infarction and each normal standard value was observed.

### INDUSTRIAL APPLICABILITY

According to the method of the present invention, cerebral infarction can be diagnosed with a high sensitivity.

Although the present invention has been described with reference to specific embodiments, various changes and modifications obvious to those skilled in the art are possible without departing from the scope of the appended claims.

## Claims

1. A method of diagnosing cerebral infarction, comprising the step of:
measuring a concentration of nicked β2 glycoprotein in a body fluid sample.

2. A method of diagnosing cerebral infarction, comprising the steps of:
measuring a nicked β2 glycoprotein I concentration (N) and a total β2 glycoprotein I concentration (T) in a body fluid sample,
calculating a ratio (N/T) of the nicked β2 glycoprotein I concentration (N) to the total β2 glycoprotein I concentration (T), and
using the ratio as an indicator.

3. The method according to claim 1 or 2, wherein the concentrations are measured by an immunological measurement or a biochemical measurement.

4. The method according to any one of claims 1 to 3, wherein at least a monoclonal antibody or an antibody fragment thereof which does not react to intact β2 glycoprotein I but reacts to nicked β2 glycoprotein I is used in the immunological measurement.

5. The method according to claim 4, wherein at least (1) a monoclonal antibody which does not react to intact β2 glycoprotein I but reacts to nicked β2 glycoprotein I and the domain V of nicked β2 glycoprotein I or (2) a monoclonal antibody which does not react to intact β2 glycoprotein I but reacts to nicked β2 glycoprotein I and a region consisting of the domains I to IV of nicked β2 glycoprotein I, is used as the monoclonal antibody.

6. The method according to any one of claims 1 to 3, wherein a heparin column is used in the biochemical measurement.

## Patentansprüche

1. Verfahren zur Diagnose eines Hirninfarkts, umfassend den Schritt:
Messen einer Konzentration von gespaltenem ("nicked") β2-Glycoprotein in einer Körperflüssigkeitsprobe.

2. Verfahren zur Diagnose eines Hirninfarkts, umfassend die Schritte:
Messen einer Konzentration an gespaltenem β2-Glycoprotein I (N) und einer Gesamtkonzentration an β2-Glycoprotein I (T) in einer Körperflüssigkeitsprobe,
Berechnen eines Verhältnisses (N/T) der Konzentration an gespaltenem β2-Glycoprotein I (N) zu der Gesamtkonzentration an β2-Glycoprotein I (T), und
Verwenden des Verhältnisses als Indikator.

3. Verfahren nach Anspruch 1 oder 2, wobei die Konzentrationen durch eine immunologische Messung oder eine biochemische Messung gemessen werden.

4. Verfahren nach einem der Ansprüche 1 bis 3, wobei mindestens ein monoclonaler Antikörper oder ein Antikörperfragment davon, welcher/welches nicht mit intaktem β2-Glycoprotein I aber mit gespaltenem β2-Glycoprotein I reagiert, in der immunologischen Messung verwendet wird.

5. Verfahren nach Anspruch 4, wobei mindestens (1) ein monoclonaler Antikörper, welcher nicht mit intaktem β2-Glycoprotein I aber mit gespaltenem β2-Glycoprotein I und der Domäne V von gespaltenem β2-Glycoprotein I reagiert, oder (2) ein monoclonaler Antikörper, welcher nicht mit intaktem β2-Glycoprotein I aber mit gespaltenem β2-Glycoprotein I und einer Region bestehend aus den Domänen I bis IV von gespaltenem β2-Glycoprotein I reagiert, als der monoclonale Antikörper verwendet wird.

6. Verfahren nach einem der Ansprüche 1 bis 3, wobei eine Heparinsäule in der biochemischen Messung verwendet wird.

## Revendications

1. Méthode de diagnostic d'un infarctus cérébral, comprenant une étape de :
mesure de la concentration de la β2 glycoprotéine clivée dans un échantillon de fluide corporel.

2. Méthode de diagnostic d'un l'infarctus cérébral, comprenant les étapes de :
mesure de la concentration (N) de la β2 glycoprotéine I clivée et de la concentration totale (T) de la β2 glycoprotéine I dans un échantillon de fluide corporel,
calcul du rapport (N/T) de la concentration (N) de la β2 glycoprotéine I clivée sur la concentration totale (T) de la β2 glycoprotéine I,
et utilisation de ce rapport comme indicateur.

3. Méthode selon la revendication 1 ou 2, dans laquelle les concentrations sont mesurées par une mesure immunologique ou une mesure biochimique.

4. Méthode selon l'une quelconque des revendications 1 à 3, dans laquelle au moins un anticorps monoclonal ou un fragment d'anticorps de celui-ci, qui ne réagit pas avec la β2 glycoprotéine I intacte mais qui réagit avec la β2 glycoprotéine I clivée est utilisé dans la mesure immunologique.

5. Méthode selon la revendication 4, dans laquelle au moins un anticorps monoclonal (1) qui ne réagit pas avec la β2 glycoprotéine I intacte mais qui réagit avec la β2 glycoprotéine I clivée et le domaine V de la β2 glycoprotéine 1 clivée ou un anticorps monoclonal (2) qui ne réagit pas avec la β2 glycoprotéine I intacte mais qui réagit avec la β2 glycoprotéine I clivée et une région consistant en les domaines I à IV de la β2 glycoprotéine I clivée, est utilisé comme l'anticorps monoclonal.

6. Méthode selon l'une quelconque des revendications 1 à 3, dans laquelle une colonne d'héparine est utilisée dans la mesure biochimique.
